(19) Europäisches Patentamt
European Patent Office
Office européen des brevets

(11) EP 0 374 808 B1

# (12) EUROPÄISCHE PATENTSCHRIFT

(45) Veröffentlichungstag und Bekanntmachung des Hinweises auf die Patenterteilung:
**17.04.1996 Patentblatt 1996/16**

(51) Int Cl.[6]: **C07D 473/06**, C07D 473/08
// C07D239/46

(21) Anmeldenummer: **89123412.2**

(22) Anmeldetag: **19.12.1989**

(54) **Neue Xanthinderivate mit Adenosin-antagonistischer Wirkung**

Xanthin derivatives having an adenosin-antagonist activity

Dérivés de xanthine à activité antagoniste d'adénosine

(84) Benannte Vertragsstaaten:
**AT BE CH DE ES FR GB GR IT LI LU NL SE**

(30) Priorität: **22.12.1988 DE 3843117**

(43) Veröffentlichungstag der Anmeldung:
**27.06.1990 Patentblatt 1990/26**

(73) Patentinhaber:
- **BOEHRINGER INGELHEIM KG**
  **D-55216 Ingelheim (DE)**
  Benannte Vertragsstaaten:
  **BE CH DE ES FR GR IT LI LU NL SE AT**
- **BOEHRINGER INGELHEIM INTERNATIONAL GmbH**
  **D-55216 Ingelheim am Rhein (DE)**
  Benannte Vertragsstaaten:
  **GB**

(72) Erfinder:
- **Küfner-Mühl, Ulrike, Dr.**
  **D-6500 Mainz (DE)**
- **Weber, Karl-Heinz, Dr.**
  **D-6535 Gau-Algesheim (DE)**
- **Walther, Gerhard, Dr.**
  **D-6530 Bingen (DE)**
- **Stransky, Werner, Dr.**
  **D-6535 Gau-Algesheim (DE)**
- **Ensinger, Helmut, Dr.**
  **D-6507 Ingelheim am Rhein (DE)**
- **Schingnitz, Günter, Dr.**
  **D-6550 Bad Kreuznach (DE)**
- **Kuhn, Franz Josef, Dr.**
  **D-6535 Gau-Algesheim (DE)**
- **Lehr, Erich, Dr.**
  **D-6531 Waldalgesheim (DE)**

(56) Entgegenhaltungen:
**GB-A- 1 201 997** **US-A- 3 624 215**
**US-A- 3 624 216** **US-A- 4 755 517**

- **CHEMICAL ABSTRACTS, Band 109, Nr. 25, 19. Dezember 1988, Seiten 19-20, Zusammenfassung Nr. 221931a, Columbus, Ohio, US; K.A. JACOBSON et al.: "8-Substituted xanthines as antagonists at A1- and A2-adenosine receptors"**
- **CHEMICAL ABSTRACTS, Band 109, Nr. 15, 10. Oktober 1988, Seite 106, Zusammenfassung Nr. 122899q, Columbus, Ohio, US; A. PATEL et al.: "125I-BW- A844U, an antogonist radioligand with high affinity and selectivity for adenosine A1 receptors, and 125I-azido-BW-A844U, a photoaffinity label"**
- **CHEMICAL ABSTRACTS, Band 108, Nr. 11, 14. März 1988, Seite 658, Zusammenfassung Nr. 94512s, Columbus, Ohio, US; M.T. SHAMIM et al.: "8-Aryl- and 8-cycloalkyl-1,3-dipropylxanthines: further potent and selective antagonists for A1-adenosine receptors"**
- **CHEMICAL ABSTRACTS, Band 107, Nr. 1, 6. Juli 1987, Seite 18, Zusammenfassung Nr. 259u, Columbus, Ohio, US; E.A. MARTINSON et al.: "Potent adenosine receptor antagonists that are selective for the A1 receptor subtype"**
- **CHEMICAL ABSTRACTS, Band 106, Nr. 19, 11. Mai 1987, Seite 53, Zusammenfassung Nr. 149315w, Columbus, Ohio, US; R.F. BRUNS et al.: "Binding of the A1-selective adenosine antagonist 8-cyclopentyl-1,3-dipropylxanthine to rat brain membranes"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 374 808 B1

- CHEMICAL ABSTRACTS, Band 88, Nr. 21, 22. Mai 1978, Seite 605, Zusammenfassung Nr. 152567v, Columbus, Ohio, US; A. ERNDT et al.: "Photochemistry of the purine system. Part II. Peroxide-initiated photoreactions of theophylline with ethers"

## Beschreibung

Die Erfindung betrifft neue Xanthinderivate, Verfahren zu ihrer Herstellung und ihre Verwendung als Arzneimittel.

Die neuen Xanthine besitzen die Struktur der allgemeinen Formel I

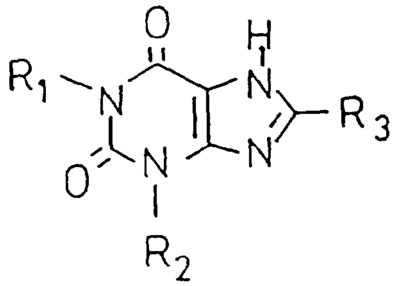

worin

$R_1$ eine Alkylgruppe mit 1 bis 4 - Kohlenstoffatomen,

$R_2$ eine Alkylgruppe mit 1 bis 4 - Kohlenstoffatomen,

$R_3$ ein durch $CONR_5R_6$ substituiertes Furan;

$R_3$ ein Tetrahydropyran-4-yl, ein Dithiolan;

$R_3$ ein $C_4$ bis $C_8$ Cycloalken, das durch $C_2$ bis $C_4$ Alkenyl substituiert sein kann,

$R_3$ ein $C_5$ Cycloalkanon oder ein $C_5$ Cycloalkanol,

$R_3$ ein - $C_5$ Cycloalkan, das gegebenenfalls durch $C_1$ bis $C_6$ - bevorzugt $C_1$ bis $C_4$ - Alkyl, $=CH_2$, -OCONH-Phenyl, $-OR_4$, $-OR_7$, $-CH_2-OH$, $-(CH_2)_l-OR_7$, bedeutet, oder einen Rest =CAH - wobei A die Bedeutung von $COOR_4$, - substituiert ist, oder das Cycloalkan durch Methyl substituiert ist und als zweiten Substituenten eine Hydroxylgruppe in geminaler Position zum ersten Substituenten trägt;

$R_3$ einen Rest der Formel

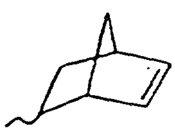 , 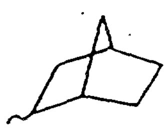 , 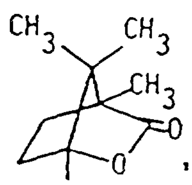 ,

und

$R_4$ Wasserstoff, eine Alkylgruppe mit 1 bis 6 Kohlenstoffatomen,

$R_5$ Wasserstoff, eine Alkylgruppe mit bevorzugt 1 bis 4 - Kohlenstoffatomen,

$R_6$ Wasserstoff, eine Alkylgruppe mit 1 bis 6 - bevorzugt 1 bis 4 - Kohlenstoffatomen, eine gegebenenfalls substituierte Benzylgruppe, einen Rest der allgemeinen Formeln $-(CH_2)_n-NR_5R_5$, ($R_5$ gleich oder verschieden) mit n = einen C-verknüpften Piperidinylrest, der gegebenenfalls durch $C_1$ bis $C_4$ Alkyl oder einen N-verknüpften Benzylrest substituiert ist

$R_7$ $CO-C_1-C_{13}$-Alkyl, bevorzugt $CO-C_2-C_4$-Alkyl, Menthoxyacetyl, über eine Carbonylgruppe verknüpfter gegebenenfalls substituiertes Benzoyl, bevorzugt Trimethoxybenzoyl, einen Rest der allgemeinen Formel CO - Pyridinyl bedeuten bedeuten können sowie gegebenenfalls deren Racemate, deren optisch aktive Verbindungen wie auch deren pharmakologisch unbedenkliche Säureadditionssalze.

Bevorzugte Verbindungen der allgemeinen Formel I sind solche worin

$R_1$ n-Propyl;

$R_2$ n-Propyl;

$R_3$ Tetrahydropyran-4-yl oder Dithiolan

$R_3$ ein Cyclopentan oder Cyclohexan, gegebenenfalls substituiert durch Methyl, wobei als geminaler Substituent eine Hydroxygruppe vorhanden sein kann;

$R_3$ ein Cyclopentan oder Cyclohexan, substituiert durch , $=CH-COOCH_3$, $=CH_2$, $-CH_2OH$, $OR_4$ mit $R_4$ = Methyl $OR_7$ worin $R_7$ $COCH_3$, $COC_2H_5$, $COC_3H_7$, CO-Phenyl oder $COCH_2$-Phenyl, gegebenenfalls substituiert, CO-Pyridyl,

$R_3$ einen Rest

$R_3$ ein Cyclopentanon mit 5 ein Cyclopentanon oder Cyclopentanol

$R_3$ Norbonan oder Norbonen

$R_4$ Wasserstoff, eine Alkylgruppe mit 1 bis 3 Kohlenstoffatomen;

$R_5$ Wasserstoff, eine Alkylgruppe mit 1 bis 3 Kohlenstoffatomen;

$R_6$ Wasserstoff, Methyl, Ethyl, Propyl, $-(CH_2)_n-NH_2$ (n=2-8), $-(CH_2)_nNEt_2$ (n=2,3) oder N-Benzyl-piperidin-4-yl-,

$R_7$ Prolinoyl, $CO-(CH_2)_{0-3}-CH_3$, (-) - Menthoxyacetyl, Benzoyl, 3,4,5-Trimethoxybenzoyl, ein Nicotinsäure-, Isonicotinsäure- oder Picolinsäurerest, N-Methylnicotinsäurerest, N-Methyl-4H-Nicotinsäurerest bedeuten können, sowie gegebenenfalls deren Säureadditionssalze.

Besonders bevorzugt sind Verbindungen der allgemeinen Formel I, worin $R_3$ einen gegebenenfalls substituierten Cyclopentanrest bildet, wobei der Substituent sich in der 3-Position des Cyclopentanringes befindet. Ganz besonders bevorzugt ist der Rest $R_3$ für 3-Oxocyclopentan.

Als Alkylgruppen werden beispielsweise - auch als Bestandteil anderer Substituenten - Methyl, Ethyl, Propyl, Isopropyl, Butyl, sek.-Butyl, iso-Butyl, tert.-Butyl, Pentyl, iso-Pentyl, neo-Pentyl, Hexyl, iso-Hexyl und als Beispiele für längerkettige Alkylreste Dekanyl, Undecanyl, Dodecanyl und Tridecanyl sowie deren Isomere genannt. Als Alkenylreste werden beispielsweise Allyl (soweit sie keine Enamine bilden), Propenyl, iso-Propenyl, Butenyl und iso-Butenyl genannt. (Et=Ethyl).

Als Cycloalkylreste werden beispielsweise Cyclopropyl, Cyclobutyl, Cyclopentyl oder Cyclohexyl bezeichnet, die durch Alkyl mit 1 bis 4 Kohlenstoffatomen substituiert sein können. Ein Benzylrest wie auch eine Phenylgruppe können ein oder mehrfach durch Alkyl mit 1 bis 4 Kohlenstoffatomen - bevorzugt Methyl-, durch Alkoxy mit 1 bis 4 Kohlenstoffatomen - bevorzugt Methoxy-, Hydroxy, und/oder Halogen - wie z.B. Fluor, Chlor, oder Brom - substituiert sein.

Als Beispiele werden genannt:

2-Chlorphenyl, 2-Bromphenyl, 3-Fluorphenyl,
2,3-Dichlorphenyl, 4-Hydroxyphenyl, 2-Methylphenyl,
4-Methylphenyl, 3-Ethylphenyl, 4-Propylphenyl,
4-Isopropylphenyl, 4-Butylphenyl, 4-tert.-Butylphenyl,
4-Pentylphenyl, 2,4-Dimethylphenyl,
2-Trifluoromethylphenyl, 3-Trifluormethylphenyl,
2-Methoxyphenyl, 4-Methoxyphenyl, 3-Ethoxyphenyl,
2-Propoxyphenyl, 4-Butoxyphenyl, 2,4-Dimethoxyphenyl,
3,4,5-Trimethoxyphenyl, 2,4-Dinitrophenyl,
4-Nitrophenyl,

Als heterocyclische Reste, die über ein Kohlenstoffatom verknüpft sein können, werden beispielsweise, Furan, 1,3-Dithiolan, genannt, wobei der Heterocyclus wie in den Definitionen angegeben substituiert, sein kann.

Als heterocyclische Reste, die über ein C-Atom verknüpft sein können und mindestens ein Stickstoffatom enthalten,

werden beispielsweise genannt: Pyridin, Pyrrol, Pyrrolin, Pyrrolidin, Piperidin, Piperazin, Morpholin, Thiomorpholin, Imidazol, Imidazolin, Imidazolidin, Pyrazol, Pyrazolin, Pyrazolidin - wobei die genannten Heterocyclen durch $C_1$-$C_4$-Alkyl substituiert sein können.

Die erfindungsgemäßen Verbindungen sind Adenosinantagonisten; sie besitzen insbesondere hohe Affinität (bis zu 1.6 nM) zum $A_1$-Rezeptor und hohe Selektivität für diesen Rezeptor-Subtyp.

Die Substanzen antagonisieren in Hippocampusschnitten die Adenosin-induzierte Unterdrückung des Summenspikes nach elektrischer Stimulation. In vivo kann im Gehirn der Ratte ein erhöhter Acetylcholingehalt festgestellt werden.

Diese Ergebnisse deuten darauf hin, daß die beschriebenen Xanthinderivate die natürliche Zellaktivität cholinerger Neurone im Gehirn verstärken und sich somit als funktionelle Cholinomimetika mit zentralem Angriff erweisen. EEG-Untersuchungen an Katzen zeigen eine deutliche Vigilanzsteigerung an.

Derartige Substanzen sind für die symptomatische Therapie degenerativer Alterserkrankungen wie Dementia senilis und Morbus Alzheimer von großem Interesse.

Die hohe Rezeptoraffinität sollte es erlauben, mit niedrigen Dosen zu therapieren, so daß kaum mit Nebenwirkungen zu rechnen ist, die nicht auf die Blockade von Adenosinrezeptoren zurückzuführen sind. Gleichermaßen sollten aufgrund der hohen $A_1$-Selektivität der Verbindungen $A_2$-abhängige Nebeneffekte ausbleiben. Über ihre Verwendung als Gerontopsychopharmaka und Nootropica hinaus könnten die beschriebenen Adenosinantagonisten zur Behandlung von Herz- und Kreislauferkrankungen von Nutzen sein.

Weitere mögliche Indikationen sind degenerative Erkrankungen wie z.B. organisches Hirnsyndrom, Parkinson, traumatische ZNS-Schädigungen, post stroke neurological deficit, respiratory depression (intoxication, post op) frühkindliches Hirntrauma.

Pharmakologische Ergebnisse sind in der Tabelle Ia dargestellt. Die Untersuchungsmethoden entsprechen denen den in den nachfolgenden Literaturzitaten angegebenen:

Lohse M.J., V. Lenschow and U. Schwabe (1984) Mol. Pharmacol. 26, 1-9;
Virus, M.R., T. Baglajewski and M. Rachelovacki (1984) Neurotiology of Agenig 5, 61-62;
Daly, J.W., W. Padgett, M.T. Shamin, P. Butts-Lamb and J. Waters (1985) J. Med. Chem. 28, 487-492;
Bruns, R.F., G.H. Lu and T.A. Pugsley (1986) Mol. Pharmacol. 29, 331-346

Tabelle Ia

| Beispiele gemäß Tabelle I | $K_i$ [nMol] ($A_1$) |
|---|---|
| 22 | $8 . 10^{-9}$ |
| 24 | $3 . 10^{-9}$ |
| 33 | $3 . 10^{-9}$ |
| 39 | $4 . 10^{-9}$ |
| 40 | $2 . 10^{-9}$ |
| 45 | $2 . 19^{-9}$ |
| 49 | $2 . 10^{-9}$ |
| 50 | $2 . 10^{-9}$ |

Die erfindungsgemäßen Verbindungen können nach an sich bekannten Analogieverfahren hergestellt werden.

Im allgemeinen werden 8 - substituierte 1,3-Dialkylxanthine durch Umsetzung von 1,3-Dialkyldiaminouracilen mit Aldehyden, Carbonsäuren oder Carbonsäurechloriden oder durch Umsetzung von 1,3-Dialkyl-6-amino-5-nitrosouracilen mit Aldehyden erhalten.

5,6-Diamino-1,3-dimethyluracil ist käuflich; mit anderen Resten substituierte Derivate werden durch Reaktion des entsprechenden Dialkylharnstoffs mit Cyanessigsäure, nachfolgende Nitrosierung und gegebenenfalls Hydrierung oder Reduktion mit Dithionit zum Diamin hergestellt (J. Org. Chem. 16, 1879 (1951) und Can. J. Chem. 46, 3413 (1968)).

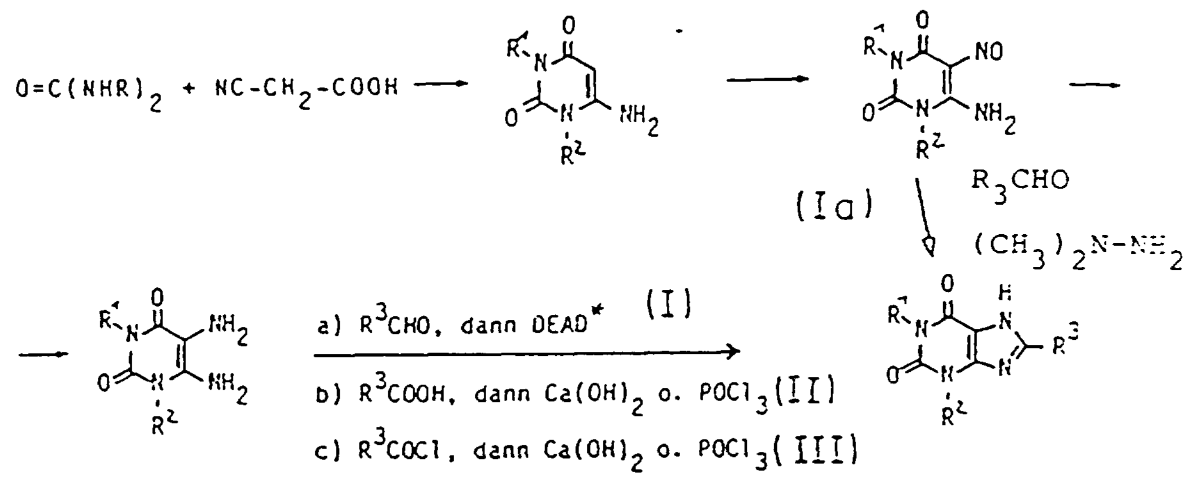


*** Diethylazodicarboxylat**

(Die römischen Ziffern nehmen bezug auf die im experimentellen Teil angegebenen Arbeitsvorschriften)

Xanthine, mit einem Benzylrest in 3-Position und einem davon verschiedenen Rest in 1-Position erhält man durch 1-Alkylierung entsprechender Vorläufermoleküle die in 3-Stellung mit einer Benzylgruppe und in 8-Stellung entsprechend substituiert sind.

Diese sind über Umsetzung von Monobenzylharnstoff und Cyanessigsäure zum 6-Amino-1-benzyluracil (L.-F. Tietze und Th. Eicher, Reakionen und Synthesen, Georg Thieme Verlag, Stuttgart 1981, S. 322), Alkylierung mit dem gewünschten Rest in 3-Position (XIV), Nitrosierung der 5-Stellung (XV) und Hydrierung zum 3-substituierten 1-Benzyl-5,6-Diaminouracil (XVI) zugänglich. Aldehyde, Carbonsäuren und Säurechloride, die zur Umsetzung mit 5,6-Diaminouracilen verwendet werden, können nach literaturbekannten Verfahren hergestellt werden.

In geeigneten Fällen können die beschriebenen Xanthine durch Umsetzung von 1.3-Dialkyl-6-chlorbarbitursäuren mit $H_2N\text{-}CH_2\text{-}R_3$, Nitrosierung und Ringschluß hergestellt werden (s. J. Med. Chem. 32, 1231 (1989).

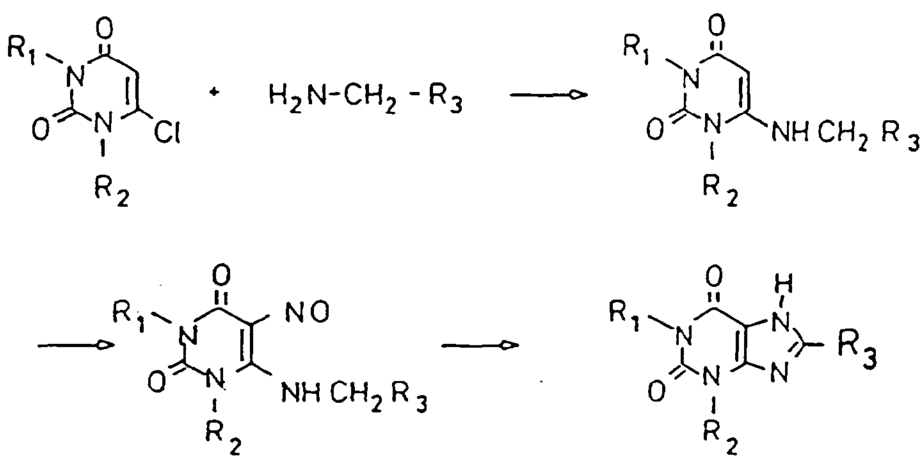

Nach den so beschriebenen Verfahren können Xanthinderivate hergestellt werden, in denen $R_3$ beispielsweise die nachfolgende Bedeutung aufweist:

Thiophen, 2-Methylthiophen, 2-Nitrothiophen Furan, Cyclohexan, Cyclohexanon, Tetrahydrofuranon, 1,3-Dithian, Pyran, Cyclobutan, Cyclohexan, Norbonen und andere, soweit die entsprechenden Aldehyde $R_3CHO$, Carbonsäuren $R_3COOH$ oder deren reaktive Derivate bereits funktionalisiert mit dem entsprechenden Diaminouracil umgesetzt werden können.

An den so erhaltenen "Xanthin-Grundkörpern" können dann weitere synthetische Variationen durchgeführt werden. Reaktive funktionelle Gruppen sind dabei ggf. auf übliche Weise zu schützen.

Ausgehend von den entsprechenden 8-Cycloalkanonen können die entsprechenden Alkohole durch Reduktion hergestellt werden, die sich wiederum mit Carbonsäuren oder Säurechloriden verestern lassen oder mit Isocyanaten zu Carbamaten umsetzen. Durch Umsetzung der entsprechenden Ketone mit Hydroxylaminen können die entsprechenden Oxime - mit substituierten Hydrazinen die entsprechenden Hydrazone erhalten werden. Die Ketofuktion kann auch auf übliche Weise mit Alkoholen ketalisiert werden. Die Reduktion der Ketale - beispielsweise mit $LiAlH_4/AlCl_3$ - ergibt die entsprechenden Ether. (In allen nachfolgenden Formeln sind deshalb die Positionen der Reste als beispielhaft zu verstehen, ohne die erfindungsgemäßen Verbindungen auf die angegebenen Positionen einzuschränken).

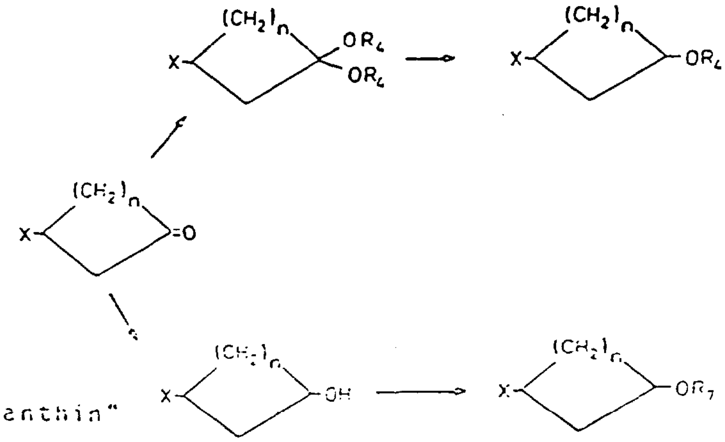

n = 1 - 5; X = "Xanthin"

Wittig-Horner-Umsetzungen an den Ketofunktionen mit Phosphonsäureestern führen zu substituierten Olefinen. Durch Veresterung der Carboxylgruppen, Amidbildung sowie Reduktion zum Alkohol und nachfolgende Veresterung oder Veretherung sind substituierte Verbindungen unten genannten Typs zugänglich, die einer nachfolgenden Hydrierung unterworfen werden können.

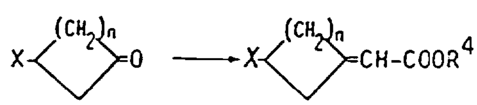

8-Furyl- können nach Vilsmeier (IV) formyliert werden. Dies so erhaltene Aldehyde dienen als Ausgangsstufen für Wittig-Horner-Reaktionen (X) mit Phosphonaten; die Produkte können entsprechend den oben angegebenen Verfahren weiter derivatisiert werden.

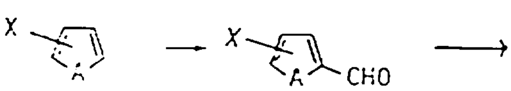

s. Folgeumsetzungen
der Wittig-Horner-
Produkte aus
Cycloalkanonen

A = O, X = Xanthin Durch Reduktion sind aus den Aldehyden die entsprechenden Alkohole zugänglich, die verestert und verethert werden können.

Oxidationsreaktionen ergeben Carbonsäuren, die ihrerseits in die Ester und Amide umgewandelt werden können.

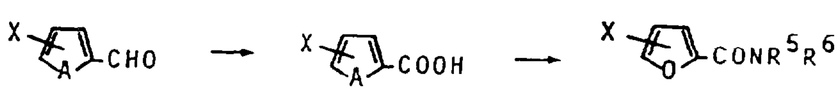

Die Doppelbindung in 8-Norbornenylderivaten kann durch Umsetzung mit $KMnO_4$ zum cis-Diol umgewandelt werden. Reaktion mit m-Chlorperbenzoesäure ergibt das Epoxid das sich um trans-Diol öffnen, mit Natriumazid zum Azidoalkohol umsetzen oder mit Lithiumtetrahydridoalanat zum entsprechenden Alkohol reduzieren läßt. Der $\alpha$-Aminoalkohol ist durch Hydrierung erhältlich.

Ausgehend von Xanthinderivaten, in denen $R_3$ ein Cycloalkanon bedeutet, erhält man durch Grignard-Reaktion oder durch Umsetzung mit Li-organischen Reagenzien Derivate der allgemeinen Formel

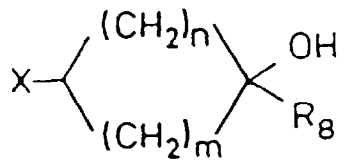

$m,n = 1,2$
$m+n = 3$

worin $R_8$ Methyl, bedeuten.

Oben genannte Cycloalkanone können mit dem sogenannten Nozaki-Lombardo Reagenz in die entsprechenden Methylenderivate

überführt werden, die anschließend zu den Methylverbindungen reduziert werden können (J. Org. Chem. 50 (8), 1212 (1985) oder nach Hydroborierung mit $BH_3$ - $CH_3SCH_3$ / $H_2O_2$, $OH^-$ die Hydroxymethylderivate ergeben.

Reduktion der Carbonylgruppe in - gegebenenfalls substituierten - Cycloalkanonen, z.B. mit Natriumtetrahydridoboranat, führt zu den entsprechenden Alkoholen die in den nachfolgenden Reaktionsschritten verestert oder verethert werden können. Die Herstellung enantiomerenreiner Xanthinderivate, die als Substituenten $R_3$ einen Cyclopentanrest tragen kann gemäß folgendem Syntheseschema erfolgen:

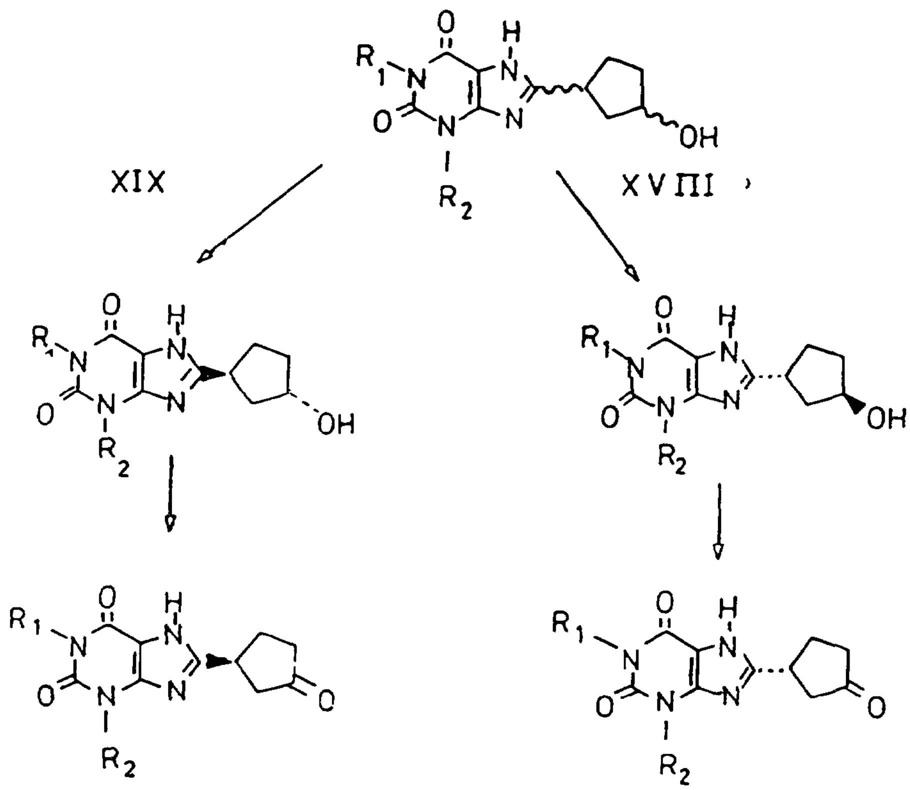


Die allgemeinen Vorschriften XVIII und XIX enthalten weitere Einzelheiten zu den stereospezifischen Synthesen.

1.3-Dipropyl-8-[3-hydroxycyclopentyl]xanthin wird mit Lipasen in organischen Lösungsmitteln enantioselektiv verestert. Durch Nachreinigung des zurückbleibenden Alkohols nach demselben Verfahren erhält man das (-)-drehende Enantiomere in über 99.5 %iger Reinheit.

Reduktive Spaltung des zuerst erhaltenen Acetats mit Lithiumaluminiumhydrid ergibt den optisch angereicherten (+)-Alkohol, der durch Umsetzung mit einer zweiten Lipase in über 99.9 %iger Entantiomerenreinheit erhalten wird. Aus diesen optisch reinen Substanzen ist eine Palette von optisch aktiven Xanthinderivaten mit substituierten Cyclopentanresten in 8-Stellung entsprechend den angegebenen Methoden zugänglich.

Geeignete erfindungsgemäße Verbindungen können nach an sich bekannten Verfahren in ihre Säureadditionssalze überführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Bevorzugte Säureadditionssalze sind die Hydrochloride sowie Hydrobromide.

Allgemeine Vorschrift I: Ringschluß mit Aldehyd

Beispiel 1

1,3-Dipropyl-8-(1,4-benzodioxan-6-yl)xanthin

2,18 g (0,013 Mol) 1,4-Benzodioxan-6-aldehyd , 80 ml Ethanol und 2,4 ml Eisessig werden gemischt und mit 2,8 g (0,012 Mol) 5,6-Diamino-1,3-dipropyluracil versetzt. Die klare Lösung wird 2 1/4 Stunden am Rückfluß gekocht und dann auf 60°C abgekühlt. Bei dieser Temperatur werden 2,1 ml (0,013 Mol) Azodicarbonsäurediethylester zugetropft, und die entstehende zähe Suspension wird mit 80 ml Ethanol versetzt und 2 Stunden am Rückfluß gekocht. Nach weiteren 20 Stunden bei Raumtemperatur wird das Gemisch auf 5°C gekühlt, der Feststoff abgesaugt und mit Ethanol und Ether gewaschen. Man erhält 4,1 g der Titelverbindung als grauen Feststoff (= 92 % d.Th), Fp=280-282°C.

Allgemeine Arbeitsvorschrift Ia: Ringschluß mit Aldehyden

Beispiel 1a

1-Propyl-3-benzyl-8-(1,4-benzodioxan-6-yl)xanthin

2,9 g (0.01 Mol) 1-Benzyl-3-propyl-5-nitroso-6-aminouracil werden mit 2,3 g (0,014 Mol) 1,4-Benzodioxan-6-aldehyd in 60 ml Dimethylformamid vorgelegt, anschließend werden 0,5 g (0,014 Mol) 1,1-Dimethylhydrazin zugegeben und die Mischung wird 8 Stunden am Rückfluß erhitzt. Nach üblicher Aufarbeitung wird der kristalline Rückstand mit Ethanol verrieben und abgesaugt. Man erhält 1.0 g der Titelverbindung als gelbe Kristalle vom Fp. = 290°C.

Allgemeine Vorschrift II: Ringschluß mit Carbonsäure

Beispiel 2

1,3-Dipropyl-8-(tetrahydropyran-4-yl)xanthin

3,2 g (0,025 Mol) Tetrahydropyran-4-carbonsäure, 4,0 g (0,025 Mol) Carbonyldiimidazol und 85 ml abs. Methylenchlorid werden 30 Minuten bei Raumtemperatur gerührt. Nach Zugabe von 5,7 g (0,025 Mol) 5,6-Diamino-1,3-dipropyluracil wird die Mischung 4 Stunden bei Raumtemperatur gerührt und dann im Vakuum eingeengt. Der Rückstand wird mit 130 ml Wasser und 11,6 g Calciumhydroxid versetzt, 30 Minuten bei 80°C gerührt und nach dem Abkühlen unter Eiskühlung mit konz. HCl sauer gestellt. Das Gemisch wird mit Ethylacetat extrahiert und die organische Phase wird getrocknet und eingeengt. Chromatographie des kristallinen Rückstands an Kieselgel ($CH_2Cl_2/CH_3OH$ 99:1) liefert 1,7 g der Titelverbindung als weiße Kristalle (15 % d. Th.), Fp. 171-172°C.

<u>Beispiel 2a</u>

1,3-Dipropyl-8-(3-oxocyclopentyl)-xanthin

2,4 g (0,014 Mol) 1,4-Dioxaspiro[4,4]nonan-7-carbonsäure werden in 56 ml Methylenchlorid gelöst und nach Zugabe von 2,2 g (0,014 Mol) Carbonyldiimidazol 1 h bei Raumtemperatur gerührt. Anschließend werden 3,2 g (0,014 Mol) 5,6-Diamino-1,3-dipropyluracil zugegeben, und man rührt weiter 4 h bei Raumtemperatur. Die Lösung wird i.V. eingeengt, der ölige Rückstand mit 70 ml Wasser und 4,5 g $Ca(OH)_2$ versetzt und 1 h bei 70°C gerührt. Man gibt 100 ml 50 %ige NaOH zu, rührt eine weitere Stunde bei 70°C und 16 h bei Raumtemperatur. Unter Eiskühlung wird die Lösung mit HCl auf pH 6 eingestellt und mit Methylenchlorid extrahiert. Die vereinigten organischen Phasen ergeben nach Trocknen und Einengen i.V. einen kristallinen Rückstand, der mit Aktivkohle aus Ethanol umkristallisiert wird. Man erhält 0,8 g (16 %) weiße Kirstalle vom Fp. 147 - 148°C.

Die Dioxolan-Schutzgruppe wird dann auf literaturbekannte Weise mit Säure hydrolysiert und man erhält die Titelverbindung.

Beispiel 2b

1,3-Dipropyl-8-(3-oxocyclopentyl)-xanthin

a) Darstellung von 3-Oxo-cyclopentancarbonsäure

100,0 g 3-Oxocyclopentancarbonsäuremethylester (0,7 Mol) werden mit 1000 ml 2-molarer Salzsäure versetzt und 10 h bei Siedetemperatur gerührt.

Die Lösung wird abgekühlt und im Vakuum vollständig eingeengt. Das restliche Wasser wird 3 x mit je 50ml Toluol abgeschleppt (Toluol zum Rückstand zugeben und am Rotavapor bei vollem Wasserstrahlvakuum und einer $H_2O$-Badtemperatur von 60° - 70°C abdestillieren). Die Rohausbeute wird im Hochvakuum fraktioniert destilliert.

1. Fraktion: $Kp_{0,02}$ 20° - 110°C (Ergab: 1,2 g Öl)
2. Fraktion: $Kp_{0,02}$ 110° - 116°C
Ergab: 4,7 g teilweise kristallines Öl
3. Franktion: $Kp_{0,002}$ 116° - 121°C
Ergab: 74,0 g farbloses Öl, kristallisierte später aus

Ausbeute 74,0 g (82,1 % d.Th.)

8,8 g 3-Oxocyclopentancarbonsäure (0,072 Mol) werden in 240 ml absolutem Methylenchlorid vorgelegt und unter Rühren werden bei 20° - 25°C 11,6 g Carbonyldiimidazol zugegeben und 2 h bei Raumtemperatur gerührt. Es werden dann 16,0 g 1,6-Diamino-1,3-di-n- propyl-uracil (0,072 Mol) bei 20° - 25°C zugegeben und weiter 3 h bei Raumtemperatur gerührt. Die Reaktionsmischung wird im Vakuum bis zur Trockne eingeengt. Der ölige Rückstand wird mit 3200 ml Wasser dest. und 35 g Calciumhydroxid versetzt und 0,5 h bei 80° C gerührt. Es wird dann auf 5°C abgekühlt und mit konz. Salzsäure auf pH 1 - 2 eingestellt und 3 x mit je 100 ml $CH_2Cl_2$ extrahiert. Die vereinigten organischen Phasen werden 1 x mit 100 ml Wasser gewaschen, mit Magnesiumsulfat getrocknet, abfiltriert und zur Trockne eingeengt. Die Rohausbeute wird über 350 g Kieselgel S160 mit ca. 41 Fließmittel $CH_2Cl_2$:$CH_3OH$ 99 : 1 gereinigt. Die sauberen Fraktionen werden bis zur Trockne eingeengt. Der kristalline Rückstand wird mit 100 ml Ether verrieben und abgesaugt.
Ausbeute: 11,5 g graue Kristalle (50,2 % d.Th.)
Fp: 164 - 168°C

Allgemeine Vorschrift III: Ringschluß mit Säurechlorid

Beispiel 3

1,3-Dipropyl-8-(4,7,7-trimethyl-2-oxa-bicyclo[2,2,1]-heptan-3-on-1-yl)xanthin

1,2 g (5,4 mMol) 5,6-Diamino-1,3-dipropyluracil und 1,0 g Triethylamin (10 mmol) werden in 50 ml abs. Methylenchlorid gelöst. Nach Zutropfen von 1,2 g (5,5 mmol) Camphansäurechlorid wird 20 Stunden bei Raumtemperatur gerührt und im Vakuum eingeengt. Der Rückstand wird mit 28 ml Wasser und 1,7 g Calciumhydroxid versetzt und 3 Stunden bei 80°C gerührt. Die abgekühlte Suspension wird unter Eiskühlung sauer gestellt und mit Methylenchlorid extrahiert. Die vereinigten organischen Phasen werden getrocknet und eingeengt, der Rückstand wird durch Chormatographie an Kieselgel ($CH_2Cl_2$/$CH_3OH$ 99:1) gereinigt.
Man erhält 200 mg der Titelverbindung als weiße Kristalle (10 % d. Th.), Fp. 200-201°C.

Allgemeine Vorschrift IV: Vilsmeier-Reaktion

Beispiel 4

1,3-Dipropyl-8-(2-formylfuran-5-yl)xanthin

Zu 400 ml absolutem Dimethylformamid werden bei 0-10°C 16,4 g (0,11 Mol) Phosphoroxychlorid zugetropft. Bei 5-15°C wird eine Lösung von 15,0 g (0,05 Mol) 1,3-Dipropyl-8-furanylxanthin in 330 ml Dimethylformamid zugegeben. Der Ansatz wird 1 Stunde bei Raumtemperatur und 7 Stunden bei 85°C gerührt. Die Mischung wird auf 500 ml Eis gegeben und mit Methylenchlorid extrahiert. Die vereinigten organischen Extrakte werden getrocknet und im Vakuum eingeengt, der Rückstand wird aus Ether kristallisiert.
Man erhält 12,1 g der Titelverbindung als braune Kristalle (73 % d. Th.), Fp. 215-217°C.

Allgemeine Vorschrift V: Oxidation eines Aldehyds zur Säure

Beispiel 5

1,3-Dipropyl-8-(2-carboxyfuran-5-yl)xanthin

Eine Lösung von 0,26 g (1,5 mmol) Silbernitrat in 2 ml Wasser wird mit einer Lösung von 0,4 g Natriumhydroxid in 1 ml Wasser 5 Minuten geschüttelt. Der grauschwarze Silberoxid-Niederschlag wird abgesaugt und mit Wasser gewaschen, anschließend in 5 ml Wasser aufgenommen und mit 1,3-Dipropyl-8-[5-formyl-(2-furanyl)]xanthin versetzt. Die Mischung wird auf 50°C erhitzt, und eine Lösung von 0,1 g Natriumhydroxid in 2 ml Wasser wird langsam zugetropft. Der Zusatz wird 15 Minuten bei 50°C und 1 Stunde bei Raumtemperatur gerührt und filtriert. Das Filtrat wird sauer gestellt und mit Methylenchlorid versetzt, der ausgefallene Niederschlag abgesaugt und mit Methylenchlorid und Ether gewaschen.
Man erhält 0,4 g der Titelverbindung als hellbraune Kristalle (77 % d. Th.)

Allgemeine Vorschrift VI: Knoevenagel-Reaktion

Beispiel 6

1,3-Dipropyl-8-[2-(2,2'-bis(ethoxycarbonyl)vinyl)-furan-5-yl]xanthin

2,5 g (7,6 mmol) 1,3-Dipropyl-8-[5-formyl-(2-furanyl)]-xanthin, 1,2 g (7,6 mmol) Malonsäurediethylester, 0,03 g (0,3 mmol) Piperidin, 0,09 g (1,5 mmol) Eisessig und 5 ml Benzol p.a. werden zusammengegeben und 6 Stunden am Wasserabscheider gekocht.
Nach dem Abkühlen wird der Ansatz mit 10 ml Toluol verdünnt, der Feststoff abgesaugt und in 100 ml warmem Methylenchlorid gelöst. Die Lösung wird filtriert, das Filtrat im Vakuum eingeengt und der Rückstand aus 2-Propanol umkristallisiert.
Man erhält 1,0 g der Titelverbindung als gelbe Kristalle (28 % d. Th.), Fp. = 220-222°C.

Allgemeine Vorschrift VII: allgemeine Darstellung von Amiden

Beispiel 7

1,3-Dipropyl-8-[2-(N,N-diethylaminocarbonyl)furan-5-yl]xanthin

1,0 g (2,9 mmol) 1,3-Dipropyl-8-[2-carboxyfuran-5-yl)]xanthin werden in absolutem Dimethylformamid gelöst und bei 0-5°C mit 0,38 g Triethylamin und 0,45 g (3,3 mmol) Isobutylchloroformat versetzt. Der Ansatz wird 2 Stunden bei 0-5°C gerührt, dann werden 0,34 g (2,9 mmol) N,N-Diethylamino-ethylamin zugegeben, und die Mischung wird ca. 12 Stunden im auftauenden Eisbad weitergerührt. Der Ansatz wird im Hochvakuum eingeengt, mit Methylenchlorid und Wasser versetzt, alkalisch gestellt und mit Methylenchlorid extrahiert. Die organischen Phasen werden jeweils verworfen, die wässerige Phase sauer gestellt und erneut extrahiert. Die vereinigten organischen Extrakte werden getrocknet, filtriert und eingeengt, der Rückstand wird aus Ethylacetat kristallisiert.
Man erhält 0,25 g der Titelverbindung als gelbliche Kristalle, Fp. 247-250°C.

Allgemeine Vorschrift VIII: Reduktion eines Ketons oder Aldehyds zum Alkohol

Beispiel 8

1,3-Dipropyl-8-(1-hydroxycyclopent-3-yl)xanthin

0,5 g (1,6 mmol) 1,3-Dipropyl-8-(1-oxo-3-cyclopentyl) xanthin, 10 ml Ethanol und 0,1 g (2,6 mmol) Natriumtetrahydridoboranat werden bei Raumtemperatur 2 1/2 Tage gerührt. Der Ansatz wird im Vakuum eingeengt und mit Wasser und Methylenchlorid versetzt, die wässerige Phase wird sauer gestellt und extrahiert. Die vereinigten organischen Extrakte werden getrocknet und im Vakuum eingeengt. Der Rückstand wird durch Chromatographie an Kieselgel in die Isomeren getrennt ($CH_2Cl_2/CH_3OH$ 95:5). Man erhält aus der 1. Fraktion: 0,4 g der Titelverbindung als weiße Kristalle (39 % d. Th) Fp. 174-176°C und aus der 2. Fraktion: 0,4 g der Titelverbindung als weiße Kristalle (39 % d. Th.) Fp. 191-193°C.

Allgemeine Vorschrift IX: Acylierung eines Alkohols

Beispiel 9

1,3-Dipropyl-8-[1-((4,7,7-trimethyl-2-oxa-bicyclo[2,2,1]heptan-3-on-1-yl)carbonyloxy)cyclopentan-3-yl]xanthin

0,2 g (0,6 mmol) 1,3-Dipropyl-8-(1-hydroxy-3(1-hydroxy-3-cyclopentyl)xanthin und 0,24 g (3 mmol) Pyridin werden in 10 ml abs. Methylenchlorid gemischt und nach Zugabe von 0,2 g (0,9 mmol) Camphansäurechlorid 4 Stunden bei Raumtemperatur gerührt. Der Ansatz wird mit Wasser versetzt und die wäßrige Phase wird abgetrennt.
Die organische Phase wird getrocknet und im Vakuum eingeengt, der Rückstand wird anschließend durch Chromatographie an Kieselgel ($CH_2Cl_2/CH_3OH$ 95:5) gereinigt.
Man erhält 50 mg der Titelverbindung als gelbliches Öl.

Allgemeine Vorschrift X: Wittig-Horner-Reaktion

Beispiel 10

1,3-Dipropyl-8-(1-cyanomethylencyclopent-3-yl)xanthin

0,28 g (1,6 mmol) Cyanmethanphosphonsäure-diethylester werden in 20 ml absolutem Benzol gelöst und mit 0,13 g (3,2 mmol) einer 60 %igen Natriumhydrid-Dispersion 5 Stunden am Rückfluß gekocht. Der Ansatz wird im Vakuum eingeengt und in Methylenchlorid und Wasser aufgenommen und anschließend sauer gestellt. Die wässrige Phase wird extrahiert und die vereinigten organischen Extrakte werden getrocknet und eingeengt. Die nachfolgende Chromatographie des Rückstands an Kieselgel ($CH_2Cl_2/CH_3OH$ 97:3) liefert 0,1 g der Titelverbindung als farbloses Öl (18 % d. Th.).

Allgemeine Vorschrift XI: Hydrierung von Doppelbindungen

Beispiel 11

1,3-Dipropyl-8-(norbornan-2-yl)xanthin

1,0 g (3,1 mmol) 1,3-Dipropyl-8-(5-norbornen-2-yl) xanthin wird in 30 ml Ethanol unter Zusatz von Palladium / Kohle unter Druck hydriert bis keine Wasserstoffaufnahme mehr zu beobachten ist. Der Katalysator wird abfiltriert, das Filtrat eingeengt und der Rückstand an Kieselgel ($CH_2Cl_2/CH_3OH$ 99:1) chromatographiert.
Man erhält 0,4 g der Titelverbindung als weiße Kristalle (39 % d. Th.), Fp. 136-138°C.

Allgemeine Vorschrift XII: Verseifung eines Esters

Beispiel 12

1,3-Dipropyl-8-(2-(2'-ethoxycarbonyl-2'-carboxyvinyl)furan-5-yl)xanthin

Zu einer Lösung von 0,8 g (1,4 mmol) Kaliumhydroxid in 20 ml Ethanol werden 3,2 g (6,8 mmol) 1,3-Dipropyl-8-[2-(2',2'-bis(ethoxycarbonyl)vinyl)-furan-5-yl]xanthin zugegeben und die Mischung 4 Stunden am Rückfluß gekocht. Nach dem Abkühlen wird mit 50 ml Wasser verdünnt und mit Methylenchlorid extrahiert. Die wässerige Phase wird unter Eiskühlung sauer gestellt, der entstehende Niederschlag abfiltriert und mit Wasser gewaschen.
Man erhält 2,2 g der Titelverbindung als gelbe Kristalle (73 % d. Th.), Fp. 252-253°C.

Allgemeine Vorschrift XIII: Reduktion eines Esters zum Alkohol

Der Ester 1,7 mmol wird in 5 ml Tetrahydrofuran gelöst und zu einer Suspension von Lithiumalanat (0,04 g, 1,1 mmol) in 5 ml Tetrahydrofuran zugetropft. Die Mischung wird 36 Stunden bei Raumtemperatur gerührt und mit gesättigter Diammoniumtartratlösung versetzt. Die wässrige Phase wird mit Ethylacetat extrahiert, die vereinigten organischen Extrakte werden getrocknet und im Vakuum eingeengt.
Das Produkt wird durch Kristallisation oder durch Chromatographie an Kieselgel gereinigt.

Allgemeine Vorschrift XIV; N-Alkylierung

Beispiel 13

1-Benzyl-3-propyl-6-aminouracil

3,0 g (0,014 Mol) 1-Benzyl-6-aminouracil werden mit 2,2 g (0,018 Mol) n-Propylbromid, 4,2 ml 15 %iger Natronlauge und 7 ml Ethanol 3 Stunden bei 70°C gerührt. Die Mischung wird auf Eis gegossen und mit Methylenchlorid extrahiert. Die organischen Phasen werden getrocknet und eingeengt. Das zurückbleibende Öl wird aus einer Mischung aus Methylenchlorid und Methanol kristallisiert.
Man erhält 1,62 g der Titelverbindung als weiße Kristalle (47 % d. Th.), Fp. 189-192°C.

Allgemeine Vorschrift XV: Nitrosierung

Beispiel 14

1-Benzyl-3-propyl-5-nitroso-6-aminouracil

2,0 g (7,7 mmol) 6-Amino-1-benzyl-3-propyluracil werden in 15 ml Wasser auf 80°C erwärmt und mit einer Lösung von 0,55 g Natriumnitrit in 3 ml Wasser versetzt. Nach Zugabe von 1 ml Eisessig fällt ein roter Feststoff aus. Der pH wird auf 4 eingestellt, die Suspension noch 30 Minuten bei 80°C gerührt. Nach dem Abkühlen werden die Kristalle abgesaugt und mit Wasser gewaschen.
Man erhält 1,9 g der Titelverbindung als rotviolette Kristalle (86 % d. Th.), Fp. 208-212°C/Zers.

Allgemeine Vorschrift XVI: Hydrierung der Nitrosoverbindung

Das 3-substituierte 6-Amino-1-benzyl-5-nitrosouracil wird in Methanol aufgenommen und nach Zugabe von Raneynickel unter Druck hydriert.

Der Katalysator wird abfiltriert, das Filtrat eingeengt und der Rückstand durch Kristallisation oder Chromatographie gereinigt.

Allgemeine Vorschrift XVII: Veretherung

Veretherungen von Alkoholen erfolgten durch Deprotonierung der Hydroxyfunktion mit einer starken Base (z.B. Natriumhydrid in Tetrahydrofuran oder Dimethylformamid, Natriumhydroxid) und Umsetzung mit einem Elektrophil der Art R-X, wobei X Halogen, Tosyl, Mesyl o.ä. sein kann.

Allgemeine Vorschrift XVIII:

Beispiel 15:

(+) 1,3-Dipropyl-8-(3-hydroxycyclopentyl)xanthin

a) 2.0 g (6.2 mmol) racemisches 1,3-Dipropyl-8-(3-hydroxycyclopentyl)xanthin )xanthin werden in 2 1 absolutem Toluol suspendiert und unter starkem Rühren mit 640 mg Acetanhydrid und 2.0 g Lipase aus Candidacylindracea versetzt. Nach 6 h bei Raumtemperatur wird das Enzym abfiltriert und mit Methanol gewaschen. Die vereinigten Filtrate werden zur Trockne i. V. eingeengt, und der Rückstand wird mit $CH_2Cl_2/CH_3OH$ 95 : 5 an Kieselgel chromatographiert.

b) Man erhält 0.6 g acetyliertes Produkt, das in 22 ml absolutem THF gelöst und nach Zugabe von 70 mg Lithiumaluminiumhydrid 2 h bei Raumtemperatur gerührt wird. Unter Eiskühlung hydrolysiert man tropfenweise mit 5 ml $H_2O$, säuert an und extrahiert mit Methylenchlorid. Die organische Phase wird getrocknet und eingeengt, der Rückstand mit $CH_2Cl_2/CH_3OH$ 95 : 5 an Kieselgel chromatographiert. Man erhält 490 mg Alkohol vom Drehwert

$[\alpha]_{20}^{D}$ = + 12° (c=0,4, Methanol).

c) Der optisch angereicherte Alkohol wird in 490 ml absolutem Methylenchlorid gelöst und mit 490 mg Acetanhydrid

und 1.5 g Lipase "Amano P" versetzt. Man rührt die Mischung 24 h bei Raumtemperatur, filtriert vom Enzym ab und engt das Filtrat i.V. zur Trockne ein. Chromatographie an Kieselgel mit $CH_2Cl_2/CH_3OH$ 95 : 5 ergibt 480 mg Alkohol der Titelverbindung vom Drehwert $[\alpha]_D^{20}$ = +18.2 (c = 0.5,$CH_3OH$); opt. Reinheit laut HPL > 99 %.

Allgemeine Vorschrift XIX:

Beispiel 16:

(-)-1,3-Dipropyl-8-(3-hydroxy-cyclopentyl)xanthin

a) 1.0 g racemisches 1,3-Dipropyl-8-(3-hydroxycyclopentyl)xanthin werden in 1 1 absolutem Toluol suspendiert und mit 320 g Acetanhydrid und 1.0 g Lipase aus Candida cylindracea 8 h bei Raumtemperatur gerührt. Man filtriert vom Enzym ab, wäscht es mit Methanol nach und engt die Filtrate i.V. zur Trockne ein. Der Rückstand wird mit $CH_2Cl_2/CH_3OH$ 95 : 5 an Kieselgel chromatographiert. Man erhält 0.45 g kristallinen Rückstand, der mit Ether verrieben und abgesaugt wird. Ergibt 350 mg Kristalle vom Drehwert $[\alpha]_D^{20}$ - 13.7 (c = 0.4, $CH_3OH$)

b) Der optisch angereicherte Alkohol wird erneut in Toluol mit 110 mg Acetanhydrid und 350 mg Lipase aus Candida cylindracea 16 h bei Raumtemperatur gerührt. Der Ansatz wird aufgearbeitet wie oben beschreiben. Ergibt: 200 mg farblose Kristalle vom Drehwert $[\alpha]_D^{20}$ = - 20.2 (c = 0.5, $CH_3OH$), laut HPLC Enantiomerenreinheit > 99.5 %.

Allgemeine Vorschrift XIX:

Beispiel 16:

(+) und (-) 1,3-Dipropyl-8-(3-oxocyclopentyl)xanthin

1.0 g optisch reiner Alkohol 28 wird in 30 ml absolutem Methylenchlorid gelöst und nach Zugabe von 1.1 g Pyridiniumchlorochromat 2.5 h bei Raumtemperatur gerührt. Die Mischung wurde zweimal mit $H_2O$ gewaschen, die Wasserphasen mit Methylenchlorid extrahiert und die vereinigten organischen Phasen getrocknet und i.V. eingedampft. Die Reinigung erfolgte durch Chromatographie an Kieselgel mit $CH_2Cl_2$ / $CH_3OH$ 99 : 1, 98 : 2 und 97 : 3.

In Abhängigkeit des eingesetzten optisch reinen Alkohols werden folgende Verbindungen erhalten.
(+) Alkohol ergibt
(-) 1,3-Dippropyl-8-(3-oxocyclopentyl)xanthin
[α]D20 - 8,3 (C = 0,5 Methanol);
(-) Alkohol ergibt
(+) 1,3-Dipropyl-8-(3-oxocyclopentyl)xanthin
$[\alpha]_D^{20}$ = + 8.0 (c = 0,5 Methanol).

Die offizielle Chemical abstract-Nomenklatur dieser Verbindungen ist: 8-(3-Oxocyclopenty1)-1,3-dipropyl-7H-purin-2,6 dion.

In Analogie zu den beschriebenen Arbeitsvorschriften oder nach bekannten Analogieverfahren können die in Tabelle I aufgeführten Verbindungen hergestellt werden.

## Tabelle I

| Nr. | $R^1$ | $R^2$ | $R^3$ | Fp (°C) |
|---|---|---|---|---|
| 11 | $n-C_3H_7$ | $n-C_3H_7$ | 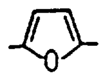 $-CONH-(CH_2)_2NEt_2$ | 247-250 |
| 12 | " | " | 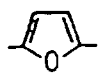 -CONH- 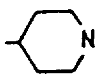 $-CH_2-$ 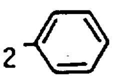 | 210-217 |

| Nr. | $R^1$ | $R^2$ | $R^3$ | Fp (°C) |
|---|---|---|---|---|
| 22 | $n-C_3H_7$ | $n-C_3H_7$ | 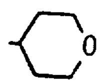 | 171-172 |

| Nr. | $R^1$ | $R^2$ | $R^3$ | Fp (°C) |
|---|---|---|---|---|
| 24 | $n-C_3H_7$ | $n-C_3H_7$ | 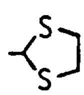 | 213-213 |

| Nr. | $R^1$ | $R^2$ | $R^3$ | Fp (°C) |
|---|---|---|---|---|
| 33 | $n-C_3H_7$ | $n-C_3H_7$ | 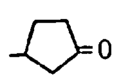 | 165-167 |
| 38 | $CH_3$ | $CH_3$ | 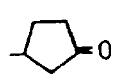 | 292 |

| Nr. | $R^1$ | $R^2$ | $R^3$ | Fp (°C) |
|---|---|---|---|---|
| 39 | $n-C_3H_7$ | $n-C_3H_7$ | 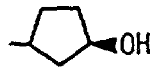 | 174-176 |
| 40 | " | " | 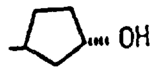 | 191-193 |
| 42 | $n-C_3H_7$ | $n-C_3H_7$ | 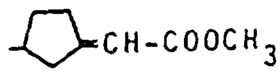 | 213-216 |
| 44 | " | " | 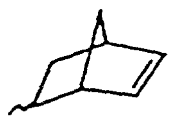 | 156-157 |
| 45 | " | " | 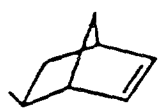 | 166-168 |
| 46 | " | " | 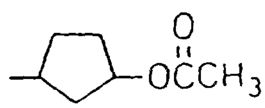 | 144 - 148 |

| Nr. | $R^1$ | $R^2$ | $R^3$ | Fp (°C) |
|---|---|---|---|---|
| 47 | " | " | 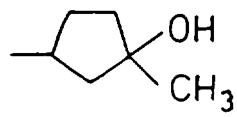 | 151-152 |
| 48 | " | " | 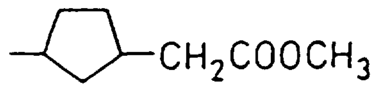 | 146-147 |
| 49 | " | " | 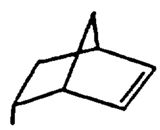 | 137-139 |
| 50 | " | " | 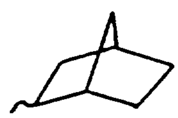 | 136-138 |
| 51 | " | " | 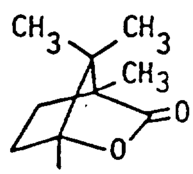 | 200-201 |
| 52 | " | " | 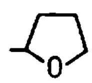 | 162 |
| 53 | " | " | 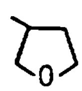 | 180 |

| Nr. | $R^1$ | $R^2$ | $R^3$ | Fp (°C) |
|---|---|---|---|---|
| 56 | " | " | 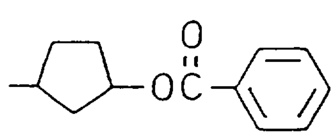 | 148-151 |

| Nr. | $R^1$ | $R^2$ | $R^3$ | Fp (°C) |
|---|---|---|---|---|
| 62 | " | " | 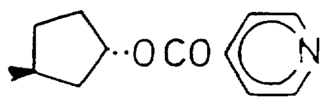 | 202-205 |
| 65 | " | " | 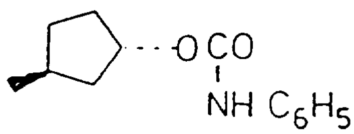 | 210-213 |
| 66 | " | " | 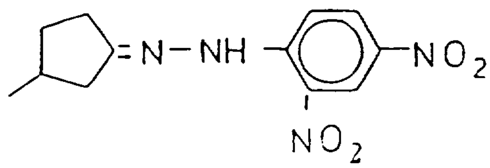 | 256-259 |
| 67 | " | " | 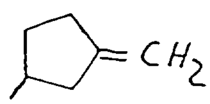 | 170-173 |

| Nr. | $R^1$ | $R^2$ | $R^3$ | Fp (°C) |
|---|---|---|---|---|
| 68 | " | " | 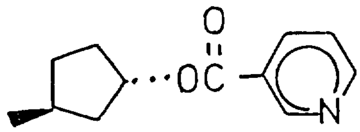 | 185-186 |
| 69 | " | " | 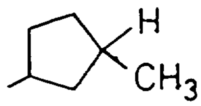 | 181-182 |
| 70 | " | " | 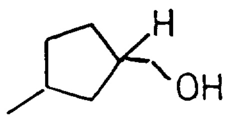 | 196-198 |
| | " | " | | 173-175 |
| 71 | " | " | 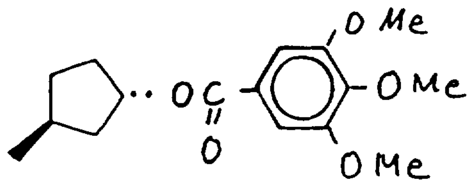 | 162-164 |
| 72 | " | " | 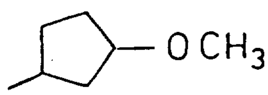 | 153-154 |
| 73 | " | " | 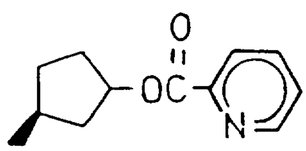 | 155-156 |
| 74 | " | " | 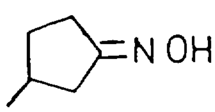 | 234-236 |

| Nr. | $R^1$ | $R^2$ | $R^3$ | Fp (°C) |
|---|---|---|---|---|
| 75 | " | " | 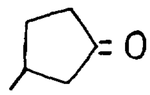 (S) | 172-173 |
| 76 | " | " | 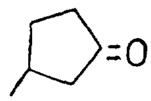 (R) | 174-175 |
| 77 | " | " | 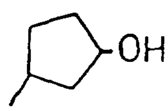 (SS) | 188-189 |
| 78 | " | " | 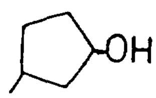 (RR) | 182-183 |

Die Verbindungen der allgemeinen Formel I können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphtalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowei ein geschmacksverbesserndes Mittel, z.B. Aromastoffe, wie Vanillin oder Orangenextrakt, enthalten.

Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Pharmazeutische Formulierungsbeispiele

| A) Tabletten | pro Tablette |
|---|---|
| Wirkstoff | 100 mg |
| Milchzucker | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet , knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) Tabletten | pro Tablette |
|---|---|
| Wirkstoff | 80 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxy-methylstärke und das Magnesiumstearat, vermischt und verpreßt das Gemisch zu Tabletten geeigneter Größe.

**Patentansprüche**

**1.** Neue Xanthine der allgemeinen Formel I

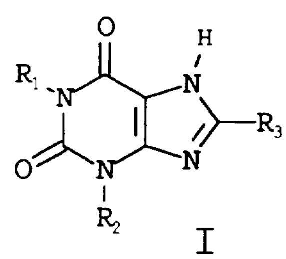

I

worin

$R_1$ eine Alkylgruppe mit 1 bis 4 - Kohlenstoffatomen;

$R_2$ eine Alkylgruppe mit 1 bis 4 - Kohlenstoffatomen;

$R_3$ ein durch $CONR_5R_6$ substituiertes Furan;

$R_3$ ein Tetrahydropyran-4-yl, ein Dithiolan;

$R_3$ ein $C_5$ - Cycloalkanon oder ein $C_5$ - Cycloalkanol;

$R_3$ ein $C_5$ - Cycloalkan, das durch $C_1$ bis $C_6$ - bevorzugt $C_1$ bis $C_4$ - Alkyl, $=CH_2$, $OR_4$, $OR_7$, $-CH_2-OH$, -OCONH-Phenyl oder =N-NH-Phenyl, wobei der Phenylrest substituiert sein kann
oder einen Rest =CAH - wobei A die Bedeutung von $COOR_4$ aufweisen kann - substituiert ist; oder das Cycloalkan durch Methyl substituiert ist und als zweiten Substituenten eine Hydroxylgruppe in geminaler Position zum ersten Substituenten tragt;

$R_3$ einen Rest der Formel

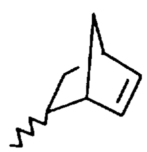
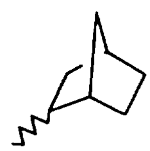
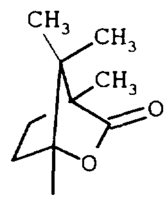

und

$R_4$ Wasserstoff, eine Alkylgruppe mit 1 bis 6 Kohlenstoffatomen;

$R_5$ Wasserstoff, eine Alkylgruppe mit 1 bis 4 Kohlenstoffatomen;

$R_6$ Wasserstoff, eine Alkylgruppe mit 1 bis 6 Kohlenstoffatomen, eine gegebenenfalls substituierte Benzylgruppe, einen Rest der allgemeinen Formel $-(CH_2)_n-NR_5R_5$, ($R_5$ gleich oder verschieden) mit n = 2, einen C-verknüpften Piperidinylrest, der gegebenenfalls durch $C_1$ bis $C_4$ Alkyl oder einen N-verknüpften Benzylrest substituiert ist;

$R_7$ $CO-C_1-C_{13}$-Alkyl, bevorzugt $CO-C_2-C_4$-Alkyl, gegebenenfalls substituiertes Benzoyl, bevorzugt Trimethoxybenzoyl, einen Rest der allgemeinen Formel CO - Pyridinyl bedeuten können, sowie gegebenenfalls deren Racemate, deren optisch aktive Verbindungen wie auch deren pharmakologisch unbedenkliche Säureadditionssalze.

**2.** Xanthine der allgemeinen Formel I, worin

$R_1$ n-Propyl;

$R_2$ n-Propyl;

$R_3$ Tetrahydropyran-4-yl oder Dithiolan

$R_3$ ein Cyclopentan substituiert durch Methyl, wobei als geminaler Substituent eine Hydroxygruppe vorhanden sein kann;

$R_3$ ein Cyclopentan substituiert durch
$=CH-COOCH_3$, $=CH_2$,
$-CH_2OH$, $OR_4$ mit $R_4$ = Methyl,
$OR_7$ worin $R_7$ $COCH_3$, $COC_2H_5$, $COC_3H_7$, CO-Phenyl,
CO-Pyridyl oder Trimethoxybenzoyl bedeutet;

$R_3$ ein Cyclopentanon oder Cyclopentanol;

$R_3$ Norbonan oder Norbonen;
sowie gegebenenfalls deren Racemate, deren optisch aktive Verbindungen wie auch deren pharmakologisch unbedenkliche Säureadditionssalze.

**3.** 1,3-Di-n-propyl-8-(3-oxocyclopentyl)-xanthin.

**4.** S-(-)-1,3-Di-n-propyl-8-(3-oxocyclopentyl)-xanthin.

**5.** 1,3-Di-n-propyl-8-(3-hydroxycyclopentyl)-xanthin.

**6.** Verfahren zur Herstellung von Xanthinen der allgemeinen Formel

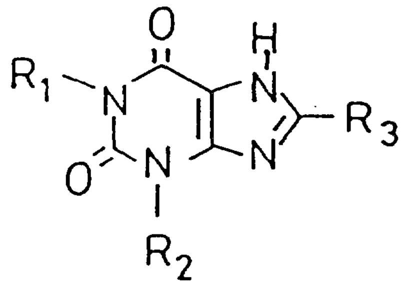

I

worin $R_1$, $R_2$ und $R_3$ wie in Anspruch 1 oder 2 definiert sind, dadurch gekennzeichnet, daß man

a) eine Verbindung der allgemeinen Formel

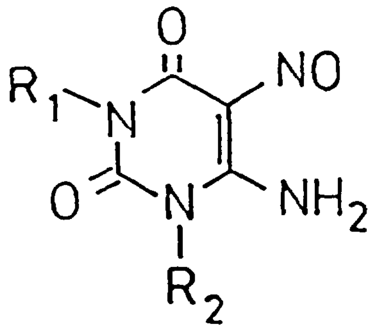

worin $R_1$ und $R_2$ wie zuvor definiert sind, mit einer Verbindung der allgemeinen Formel $R_3$ CHO - worin $R_3$ wie zuvor definiert ist, wobei funktionelle Gruppen in $R_3$ gegebenenfalls geschützt werden müssen - umsetzt und anschließend mit N,N-Dimethylhydrazin cyclisiert; oder

b) eine Verbindung der allgemeinen Formel

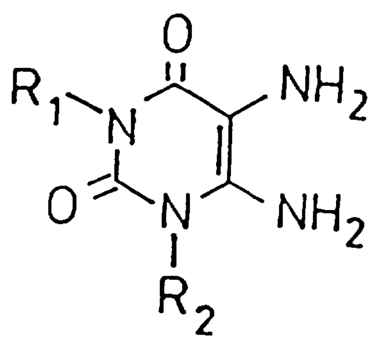

mit einer Verbindung der allgemeinen Formel $R_3CHO$, $R_3COOH$ oder deren reaktives Derivat - wobei funktionelle Gruppen in $R_3$ gegebenenfalls geschützt werden müssen - umsetzt und anschließend zu dem Xanthin der allgemeinen Formel I cyclisiert; oder

c) eine Verbindung der allgemeinen Formel

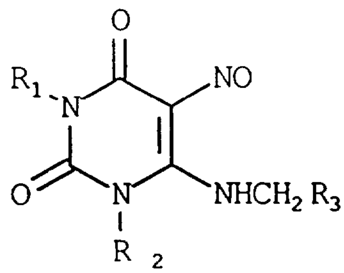

worin $R_1$, $R_2$ und $R_3$ wie zuvor definiert sind, zu einer Verbindung der allgemeinen Formel I cyclisiert, und anschließend eine nach a, b oder c hergestellte Verbindung gegebenenfalls nach an sich bekannten Verfahren wie folgt weiterbehandelt:

d) eine Verbindung der allgemeinen Formel I

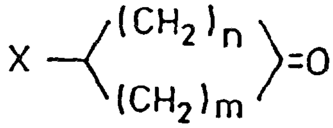

**n = 2, m = 1**

worin X den Xanthinrest bedeutet,

1) in eine Verbindung der Formel bzw.

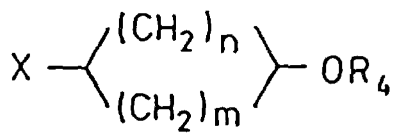

oder

2) in eine Verbindung

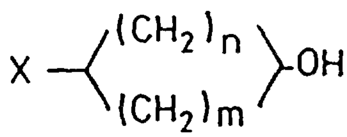

bzw.

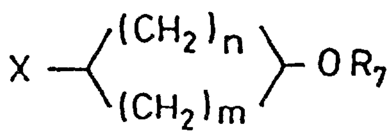

oder

3) in eine Verbindung

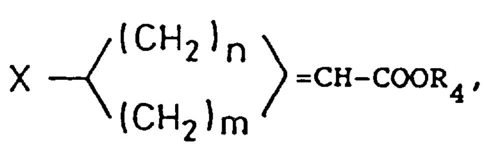

oder

4) durch Gridnard Reaktion oder durch Umsetzung mit Li-organischen Reagenzien in eine Verbindung der allgemeinen Formel

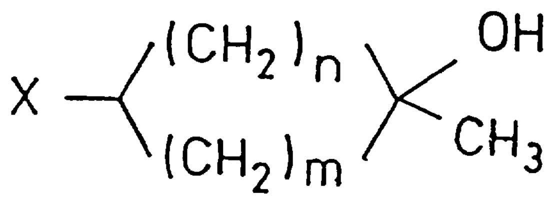

oder

5) durch Umsetzung mit dem Nozaki-Lombardo Reagenz in eine Verbindung

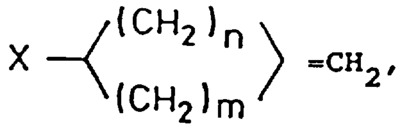

die zum Methylderivat reduziert oder mit $BH_3$ / $CH_3SCH_3/H_2O_2/OH^-$ in die entsprechende Hydroxymethylverbindung überführt;
oder

e) eine Verbindung der allgemeinen Formel

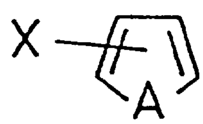

worin X den Xanthinrest und A Sauerstoff bedeuten, formyliert und

f) einen Aldehyd der Formel

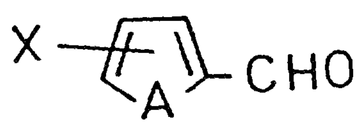

in das Amid der Formel

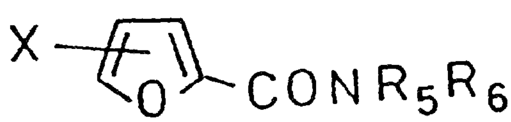

überführt,
wobei bei den oben angegebenen Umsetzungen die Reste $R_4$, $R_5$, $R_6$ und $R_7$ wie in Anspruch 1 oder 2 definiert sind;

g) geeignete Verbindungen der allgemeinen Formel I können nach an sich bekannten Verfahren in ihre Säureadditionssalze überführt werden.

**7.** Verbindung der allgemeinen Formel I gemäß Anspruch 1, 2, 3, 4 oder 5 zur Anwendung als Arzneimittel.

**8.** Verwendung einer Verbindung der allgemeinen Formel I gemäß Anspruch 1, 2, 3, 4 oder 5 zur Herstellung eines Arzneimittels mit adenosinantagonistischer Wirkung.

**9.** Pharmazeutische Zubereitungen, enthaltend als Wirkstoff eine oder mehrere Verbindungen der allgemeinen Formel I oder deren physiologisch verträgliche Säureadditionssalze in Kombination mit üblichen Hilfs- und/oder Trägerstoffen.

**10.** Verfahren zur Herstellung von pharmazeutischen Präparaten gemäß Anspruch 6, dadurch gekennzeichnet, daß man Verbindungen der allgemeinen Formel I mit üblichen galenischen Hilfs- und/oder Trägerstoffen zu üblichen

pharmazeutischen Anwendungsformen verarbeitet.

**Claims**

**1.** New xanthines of general formula I

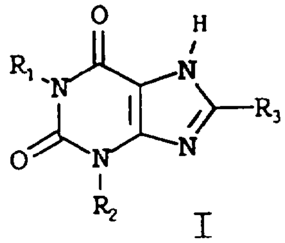

I

wherein

$R_1$ denotes a $C_{1-4}$-alkyl group;

$R_2$ denotes a $C_{1-4}$-alkyl group;

$R_3$ denotes a furan substituted by $CONR_5R_6$;

$R_3$ denotes a tetrahydropyran-4-yl, a dithiolane;

$R_3$ denotes a $C_5$-cycloalkanone or a $C_5$-cycloalkanol;

$R_3$ denotes a $C_5$-cycloalkane which is substituted by $C_{1-6}$-, preferably $C_{1-4}$-, alkyl, $=CH_2$, $OR_4$, $OR_7$, $-CH_2-OH$, -OCONH-phenyl or =N-NH-phenyl, whilst the phenyl group may be substituted, or a group =CAH (wherein A may denote $COOR_4$) ; or the cycloalkane is substituted by methyl, and contains as a second substituent a hydroxyl group in the geminal position relative to the first substituent;

$R_3$ denotes a group of the formula

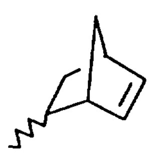

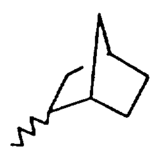

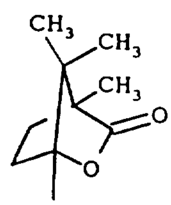

and

$R_4$ denotes hydrogen, a $C_{1-6}$-alkyl group;

$R_5$ denotes hydrogen, a $C_{1-4}$-alkyl group;

$R_6$ denotes hydrogen, a $C_{1-6}$-alkyl group, an optionally substituted benzyl group, a group of general formula $-(CH_2)_n-NR_5R_5$ (wherein $R_5$ may be identical or different) with n = 2, a C-linked piperidinyl group which is optionally substituted by $C_{1-4}$-alkyl or by an N-linked benzyl group;

$R_7$ denotes $CO-C_{1-13}$-alkyl, preferably $CO-C_{2-4}$-alkyl, optionally substituted benzoyl, preferably trimethoxybenzoyl, a group of general formula CO - pyridinyl, and optionally the racemates, the optically active compounds and the pharmacologically acceptable acid addition salts thereof.

**2.** Xanthines of general formula I, wherein

$R_1$ denotes n-propyl;

$R_2$ denotes n-propyl;

$R_3$ denotes tetrahydropyran-4-yl or dithiolane

$R_3$ denotes a cyclopentane substituted by methyl, whilst a hydroxy group may be present as a geminal substituent;

$R_3$ denotes a cyclopentane substituted by
$=CH-COOCH_3$, $=CH_2$,
$-CH_2OH$, $OR_4$ wherein $R_4$ = methyl,
$OR_7$ wherein $R_7$ denotes $COCH_3$, $COC_2H_5$, $COC_3H_7$,
CO-phenyl, CO-pyridyl or trimethoxybenzoyl;

$R_3$ denotes a cyclopentanone or cyclopentanol;

$R_3$ denotes norbornane or norbornene;
and optionally the racemates, the optically active compounds and the pharmacologically acceptable acid addition salts thereof.

**3.** 1,3-Di-n-propyl-8-(3-oxocyclopentyl)-xanthine.

**4.** S-(-)-1,3-Di-n-propyl-8-(3-oxocyclopentyl)-xanthine.

**5.** 1,3-Di-n-propyl-8-(3-hydroxycyclopentyl)-xanthine.

**6.** Process for preparing xanthines of general formula

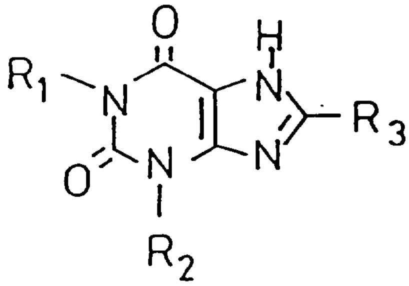

I

wherein $R_1$, $R_2$ and $R_3$ are defined as in claim 1 or 2, characterised in that

a) a compound of general formula

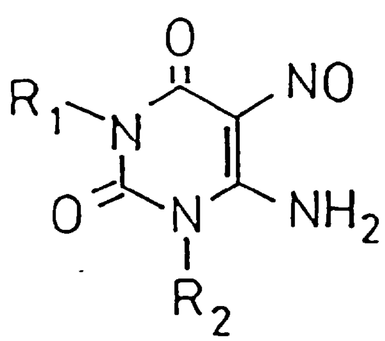

wherein $R_1$ and $R_2$ are as hereinbefore defined, is reacted with a compound of general formula $R_3CHO$ - wherein $R_3$ is as hereinbefore defined, whilst any functional groups present in $R_3$ must optionally be protected - and is subsequently cyclised with N,N-dimethylhydrazine; or

b) a compound of general formula

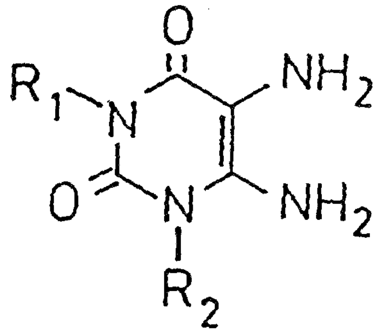

is reacted with a compound of general formula $R_3CHO$, $R_3COOH$ or a reactive derivative thereof - whilst any functional groups present in $R_3$ must optionally be protected - and subsequently cyclised to form the xanthine of general formula I; or

c) a compound of general formula

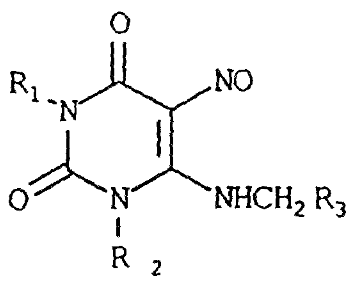

wherein $R_1$, $R_2$ and $R_3$ are as hereinbefore defined, is cyclised to form a compound of general formula I, and subsequently a compound prepared according to a, b or c is optionally further treated according to methods known per se, as follows:

d) a compound of general formula I

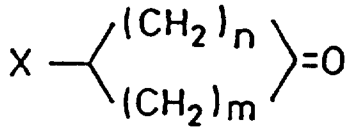

n = 2, m = 1

wherein X denotes the xanthine group,
is converted

1) into a compound of formula

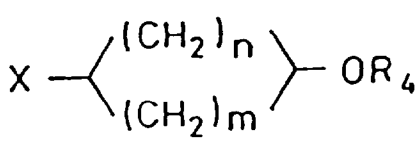

or

2) into a compound

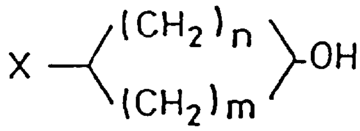

or

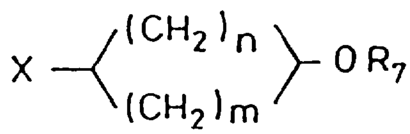

or

3) into a compound

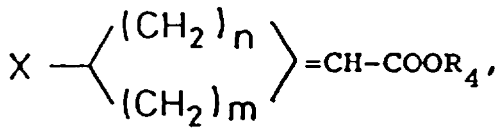

or

4) by Grignard reaction or by reacting with Li-organic reagents, into a compound of general formula

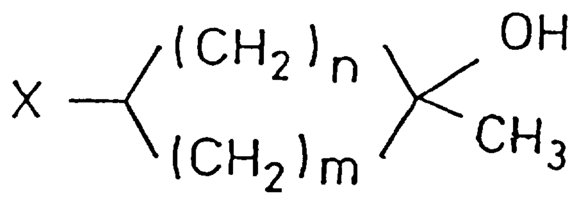

or

5) by reacting with the Nozaki-Lombardo reagent into a compound

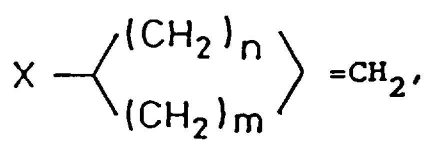

which is reduced to form the methyl derivative or with $BH_3/CH_3SCH_3/H_2O_2/OH^-$ is converted into the corresponding hydroxymethyl compound;
or

e) a compound of general formula

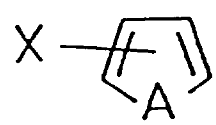

wherein X denotes the xanthine group and A is oxygen, is formylated and

f) an aldehyde of the formula

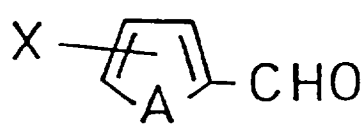

is converted into the amide of formula

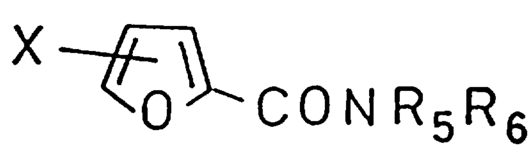

whilst in the above reactions the groups $R_4$, $R_5$, $R_6$ and $R_7$ are defined as in claim 1 or 2;

h) suitable compounds of general formula I may be converted into the acid addition salts thereof using methods known per se.

**7.** Compound of general formula I according to claim 1, 2, 3, 4 or 5 for use as a pharmaceutical composition.

**8.** Use of a compound of general formula I according to claim 1, 2, 3, 4 or 5 for preparing a pharmaceutical composition with an adenosine-antagonistic activity.

**9.** Pharmaceutical preparations containing as active substance one or more compounds of general formula I or the physiologically acceptable acid addition salts thereof combined with conventional excipients and/or carriers.

**10.** Process for preparing pharmaceutical preparations according to claim 6, characterised in that compounds of general formula I are processed with conventional galenic excipients and/or carriers to form conventional pharmaceutical preparations.

## Revendications

**1.** Nouvelles xanthines de formule générale I

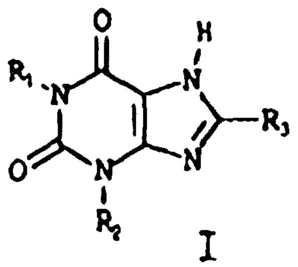

I

dans laquelle

$R_1$ peut représenter un groupe alkyle de 1 à 4 atomes de carbone;
$R_2$ peut représenter un groupe alkyle de 1 à 4 atomes de carbones ;
$R_3$ peut représenter un furane substitué par $CONR_5R_6$;
$R_3$ peut représenter un tétrahydropyran-4-yle, un dithiolane;
$R_3$ peut représenter une cycloalcanone en $C_5$ ou un cycloalcanol en $C_5$;
$R_3$ peut représenter un cycloalcane en $C_5$ qui est substitué par alkyle en $C_1$ à $C_6$, de préférence en $C_1$ à $C_4$, $=CH_2$, $OR_4$, $OR_7$, $-CH_2-OH$, -OCONH-phényle ou =N-NH-phényle, le reste phényle pouvant être substitué, ou par un reste =CAH où A peut avoir la signification de $COOR_4$; ou bien le cycloalcane est substitué par méthyle et porte comme second substituant un groupe hydroxyle en position géminée par rapport au premier substituant;
$R_3$ peut représenter un reste de formule

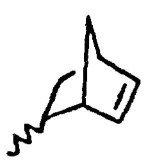

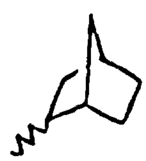

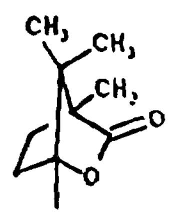

et
$R_4$ peut représenter l'hydrogène, un groupe alkyle de 1 à 6 atomes de carbone;
$R_5$ peut représenter l'hydrogène, un groupe alkyle de 1 à 4 atomes de carbone;
$R_6$ peut représenter l'hydrogène, un groupe alkyle de 1 à 6 atomes de carbone, un groupe benzyle éventuellement substitué, un reste de formule générale $-(CH_2)_n-NR_5R_5$ ($R_5$ identiques ou différents) avec n = 2, un reste pipéridinyle lié à C, qui est éventuellement substitué par alkyle en $C_1$ à $C_4$ ou par un reste benzyle lié à N;
$R_7$ peut représenter CO-alkyle en $C_1$-$C_{13}$, de préférence CO-alkyle en $C_2$-$C_4$, benzoyle éventuellement substitué, de préférence triméthoxybenzoyle, un reste de formule générale CO-pyridinyle,

ainsi que, éventuellement, leurs racémates, leurs composés optiquement actifs et aussi leurs sels d'addition d'acide irréprochables du point de vue pharmacologique.

**2.** Xanthines de formule générale I dans laquelle

$R_1$ peut représenter n-propyle;
$R_2$ peut représenter n-propyle;
$R_3$ peut représenter tétrahydropyran-4-yle ou dithiolane;
$R_3$ peut représenter un cyclopentane substitué par méthyle, un groupe hydroxyle pouvant être présent comme substituant géminé;
$R_3$ peut représenter un cyclopentane substitué par
$=CH-COOCH_3$, $=CH_2$,
$-CH_2OH$, $OR_4$ avec $R_4$ = méthyle,
$OR_7$ où $R_7$ représente $COCH_3$, $COC_2H_5$, $COC_3H_7$, CO-phényle, CO-pyridyle ou triméthoxybenzoyle;
$R_3$ peut représenter une cyclopentanone ou un cyclopentanol;
$R_3$ peut représenter norbornane ou norbornène;

ainsi que, éventuellement, leurs racémates, leurs composés optiquement actifs et aussi leurs sels d'addition d'acide irréprochables du point de vue pharmacologique.

**3.** 1,3-di-n-propyl-8-(3-oxocyclopentyl)xanthine.

**4.** S-(-)-1,3-di-n-propyl-8-(3-oxocyclopentyl)xanthine.

**5.** 1,3-di-n-propyl-8-(3-hydroxycyclopentyl)xanthine.

**6.** Procédé de préparation de xanthines de formule générale

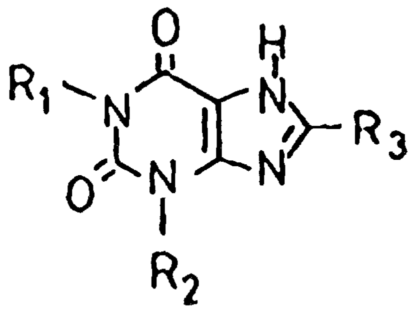

I

dans laquelle $R_1$, $R_2$ et $R_3$ sont définis comme dans la revendication 1 ou 2, caractérisé en ce que

a) on fait réagir un composé de formule générale

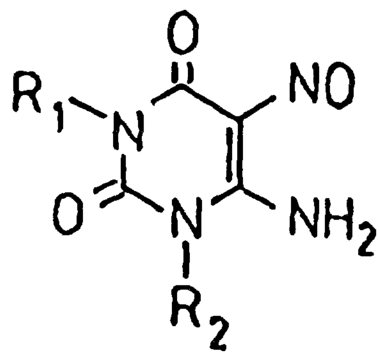

dans laquelle $R_1$ et $R_2$ sont définis comme précédemment, avec un composé de formule générale $R_3CHO$ dans laquelle $R_3$ est défini comme précédemment, des groupes fonctionnels dans $R_3$ devant éventuellement être protégés, puis on le cyclise avec la N,N-diméthylhydrazine;
ou

b) on fait réagir un composé de formule générale

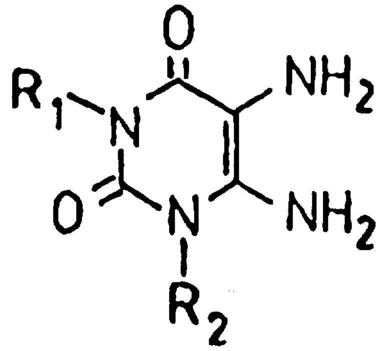

avec un composé de formule générale $R_3CHO$, $R_3COOH$ ou son dérivé réactif, des groupes fonctionnels dans $R_3$ devant éventuellement être protégés, puis on le cyclise en la xanthine de formule générale I;
ou
c) on cyclise un composé de formule générale

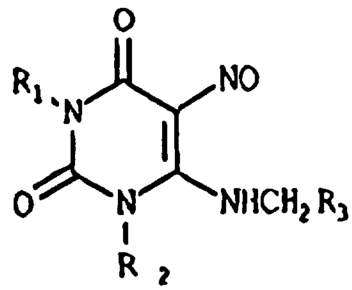

dans laquelle $R_1$, $R_2$ et $R_3$ sont définis comme précédemment, en un composé de formule générale I,
puis, éventuellement, on soumet à un traitement ultérieur de la manière suivante, selon des procédés connus en soi, un composé préparé selon a, b ou c:
d) on convertit un composé de formule générale I

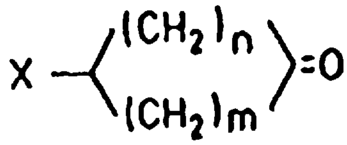

**n = 2, m = 1**

dans laquelle X représente le reste xanthine,

1) en un composé de formule

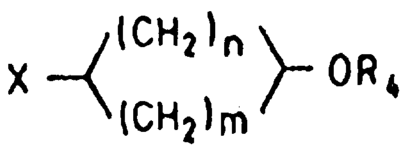

ou
2) en un composé

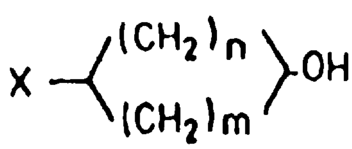

ou

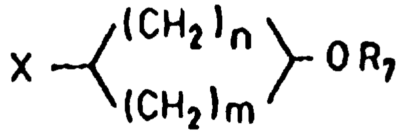

ou

3) on un composé

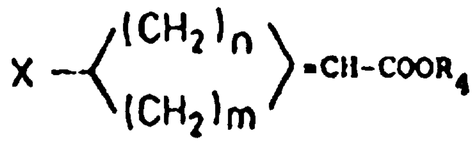

ou

4) par réaction de Grignard ou par réaction avec des réactifs Li-organiques en un composé de formule générale

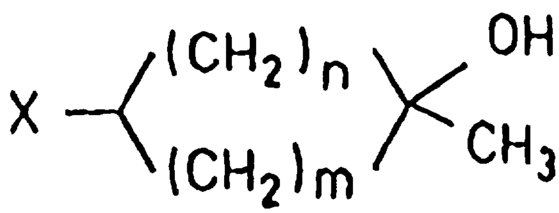

ou

5) par réaction avec le réactif de Nozaki-Lombardo en un composé

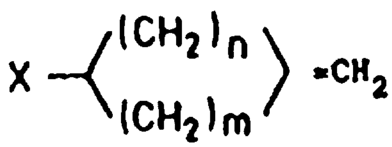

qui est réduit en le dérivé méthylé ou converti avec $BH_3/CH_3SCH_3/H_2O_2/OH^-$ en le composé hydroxyméthylé correspondant;

ou

e) on formyle un composé de formule générale

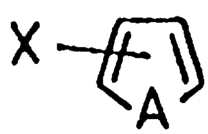

dans laquelle X représente le reste xanthine et A représente l'oxygène et

f) on convertit un aldéhyde de formule

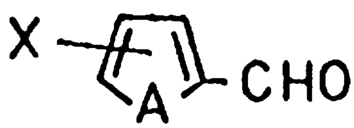

en l'amide de formule

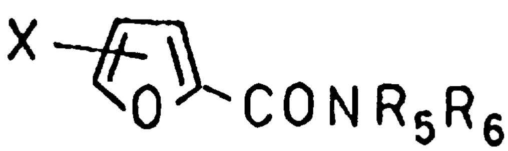

dans les réactions indiquées ci-dessus, les restes $R_4$, $R_5$, $R_6$ et $R_7$ étant définis comme dans la revendication 1 ou 2; h) des composés de formule générale I appropriés peuvent être convertis en leurs sels d'addition d'acide selon des procédés connus en soi.

**7.** Composé de formule générale I selon la revendication 1, 2, 3, 4 ou 5 destiné à être utilisé comme médicament.

**8.** Utilisation d'un composé de formule générale I selon la revendication 1, 2, 3, 4 ou 5 pour la préparation d'un médicament à effet antagoniste de l'adénosine.

**9.** Préparations pharmaceutiques contenant comme principe actif un ou plusieurs composés de formule générale 1 ou leurs sels d'addition d'acide physiologiquement acceptables en combinaison avec des adjuvants et/ou supports habituels.

**10.** Procédé d'obtention de préparations pharmaceutiques selon la revendication 6, caractérisé en ce que l'on transforme des composés de formule générale I en formes d'utilisation pharmaceutiques habituelles avec des adjuvants et/ou supports galéniques habituels.